(19) Europäisches Patentamt
European Patent Office
Office européen des brevets

(11) Veröffentlichungsnummer: **0 004 975**
**B1**

# (12) EUROPÄISCHE PATENTSCHRIFT

(45) Veröffentlichungstag der Patentschrift: **17.02.82**

(51) Int. Cl.³: **C 07 J 7/00,** A 61 K 31/57 // C07J21/00, C07J31/00

(21) Anmeldenummer: **79101200.8**

(22) Anmeldetag: **20.04.79**

(54) **Corticoid-17-Alkylcarbonate, Verfahren zu deren Herstellung sowie deren Verwendung als Arzneimittel gegen Dermatosen.**

(30) Priorität: **25.04.78 DE 2817988**

(43) Veröffentlichungstag der Anmeldung: **31.10.79 Patentblatt 79/22**

(45) Bekanntmachung des Hinweises auf die Patenterteilung: **17.02.82 Patentblatt 82/7**

(84) Benannte Vertragsstaaten: **BE CH DE FR GB IT LU NL SE**

(56) Entgegenhaltungen:
**EP-A-0 000 742**
**DE-A-1 902 340**
**LU-A-68 212**
**US-A-3 558 675**
**US-A-4 021 459**

(73) Patentinhaber: **HOECHST AKTIENGESELLSCHAFT, Postfach 80 03 20, D-6230 Frankfurt/Main 80 (DE)**

(72) Erfinder: **Stache, Ulrich, Dr., Goldgraben 20, D-6238 Hofheim am Taunus (DE)**
Erfinder: **Fritsch, Werner, Dr., Fuchshohl 1, D-6232 Bad Soden am Taunus (DE)**
Erfinder: **Alpermann, Hans Georg, Dr., Am Eichkopf 10, D-6240 Königstein/Taunus (DE)**
Erfinder: **Sandow, Jürgen Kurt, Dr., Am Haideplacken 22, D-6240 Köningstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 975 B1

# Corticoid-17-Alkylcarbonate, Verfahren zu deren Herstellung sowie deren Verwendung als Arzneimittel gegen Dermatosen

Gegenstand der Erfindung sind neue Steroid-21-halogen-17-alkylcarbonate der Formel I oder II

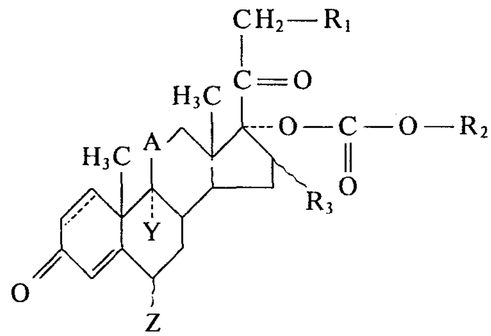

(I)

oder

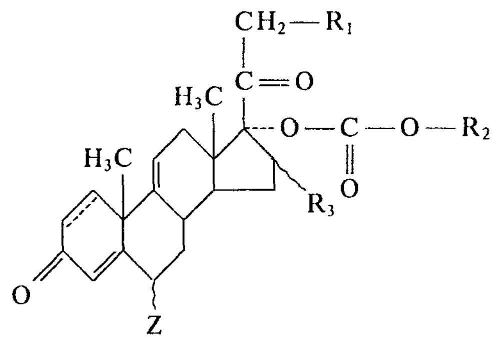

(II)

in der A die Gruppierungen

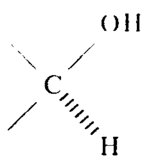

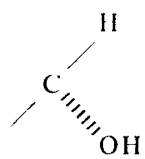

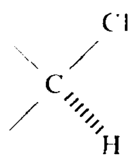

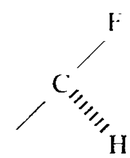

C=O

Y Wasserstoff, Fluor oder Chlor,
Z Wasserstoff, Chlor, Fluor oder Methyl,
$R_1$ Fluor, Chlor, Brom, Jod,
$R_2$ verzweigtes oder unverzweigtes Alkyl mit 1 bis 10 C-Atomen,
$R_3$ Wasserstoff, $\alpha$- oder $\beta$-ständiges Methyl, Fluor oder eine gegebenenfalls durch ein oder zwei Fluoratome substituierte Methylengruppe

bedeuten, wobei in 1,2-Stellung eine zusätzliche Doppelbindung vorhanden sein kann.

Aus der deutschen Offenlegungsschrift 1 902 340 sind Steroid-Verbindungen bekannt, die sich von vorliegenden dadurch unterscheiden, daß sie in 17-Stellung eine Acyloxygruppe enthalten, während die erfindungsgemäßen Verbindungen an dieser Stelle eine Alkylcarbonatgruppe tragen. Die erfindungsgemäßen Verbindungen zeichnen sich gegenüber den bekannten durch eine verbesserte antiphlogistische Wirksamkeit aus.

In der luxemburgischen Patentschrift 68 212 sind Verbindungen beschrieben, die sich von den erfindungsgemäßen dadurch unterscheiden, daß sie in 21-Stellung eine freie Hydroxylgruppe tragen. Derartige Verbindungen sind gegen Säuren instabil. Sie erfahren in saurem bis neutralem Milieu eine Umlagerung zu den entsprechenden 21-Alkylcarbonaten mit freier 17-Hydroxylgruppe.

Endlich sind aus der US-Patentschrift 3 558 675 Steroid-Carbonate bekannt, die sich von den erfindungsgemäßen Substanzen durch unterschiedliche Substitution in 11- und/oder 21-Stellung unterscheiden. Diese bekannten Verbindungen besitzen eine Gestagen-Hormonwirkung und unterscheiden sich dadurch in ihrer Wirkungsweise grundlegend von den erfindungsgemäßen Substanzen, die antiinflammatorische und antiphlogistische Wirksamkeit besitzen.

Gegenstand der Erfindung ist ferner ein Verfahren zur Herstellung von Verbindungen der Formel I oder II, das dadurch gekennzeichnet ist, daß man

a) ein Steroid-21-alkyl- bzw. arylsulfonat-17-alkylcarbonat der Formel I oder II, in der $R_2$, $R_3$, A, Y und Z die genannte Bedeutung haben, $R_1$ jedoch einen Rest der Formel III

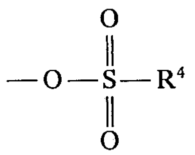

(III)

darstellt, worin $R^4$ $C_1-C_4$-Alkyl, Phenyl, Methylphenyl-, Äthylphenyl, Fluor-, Brom- oder Chlorphenyl oder Nitrophenyl bedeutet,
mit einer Halogen-Anionen liefernden Verbindung in Gegenwart von inerten organischen Lösungsmitteln umsetzt, wobei gegebenenfalls nach dem Phasentransferverfahren auch wäßrige Medien benutzt werden können, oder

b) ein Corticosteroid-17,21-dialkylorthocarbonat der Formel IV oder V

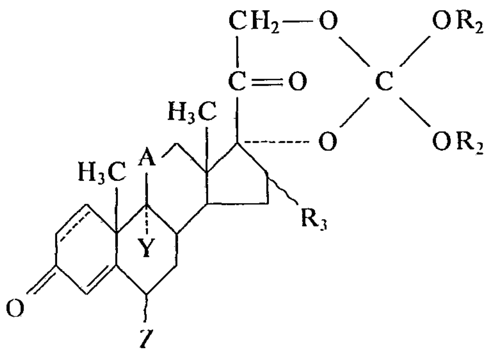

(IV)

oder

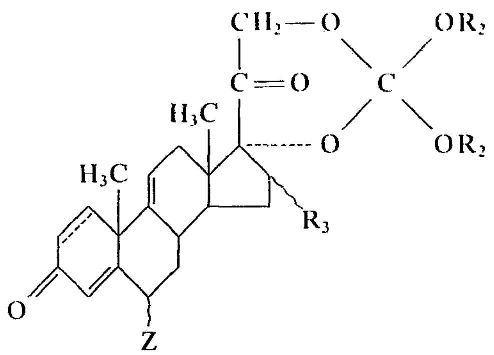

(V)

in der A, Y, Z, $R_2$ und $R_3$ die angegebene Bedeutung haben, mit Halogen abgebenden anorganischen oder organischen Säurehalogenid-Verbindungen, oder mit Triphenylmethylchlorid in inerten organischen Lösungsmitteln umsetzt.

Die als Ausgangssubstanzen benötigten Steroid-21-alkyl- bzw. arylsulfonat-17-alkylcarbonate der Formel I oder II, in der $R_1$ einen Alkyl-, Aralkyl- oder gegebenenfalls substituierten Arylsulfonsäureesterrest bedeutet, sind gemäß der deutschen Offenlegungsschrift (DOS) 2 735 110 (HOE 77/F 154) herstellbar. Die für die Verfahrensvariante b) als Ausgangssubstanzen benötigten Steroid-17α,21-dialkyl-orthocarbonate der allgemeinen Formel IV oder V sind in der Regel bekannt und können nach der deutschen Patentschrift DBP 1 668 079 hergestellt werden.
Es kommen die Steroid-21-alkyl- oder arylsulfonat-17-alkylcarbonate bzw. Steroid-17α,21-dialkylorthocarbonate folgender 17,21-Dihydroxy-steroide bzw. -corticoide in Betracht:
Cortison, Hydrocortison, Reichsteins Substanz S, Prednison, Prednisolon, 6α-Methylprednisolon, 16α- oder 16β-Methylprednisolon, 9α-Fluor- oder 9α-Chlor-prednisolon, 16-Methylenprednisolon, 6α,9α-Difluorprednisolon, 6α-Methyl-9α-fluor-prednisolon, 6α-Fluor-prednisolon, 9α-Fluor-16α-methyl-prednisolon, 9α-Fluor-prednisolon, 9α-Fluor-16α-methyl-prednisolon, 9α-Fluor-prednisolon, 9α-Fluor-16α-methyl-prednisolon, 6α-Fluor-16α-methyl-prednisolon, 6α-Fluor-16β-methyl-prednisolon, 6α-Fluor-16-methylen-prednisolon, 6α,9α-Difluor-16α-methyl-prednisolon, 6α,9α-Difluor-16β-methyl-prednisolon, 6α,9α-Difluor-16-methylen-prednisolon, 9α-Fluor-6α,16α-dimethylprednisolon, 9α,16α-Difluor-prednisolon, 6α,9α-Trifluor-prednisolon, 17α,21-Dihydroxy-$\Delta^{4(5),9(11)}$-pregnadien-dion-(3,20), 17α,21-Dihydroxy-9α, 11-β-dichlor-$\Delta^{1,4}$-pregnadien-dion-(3,20), Desoxycorticosteron, Corticosteron, 16α-Methyl-corticosteron, 9α-Fluor-16α-methyl-corticosteron, 6α,9α-Difluor-16α-methyl-corticosteron, 6α-Fluor-16α-methyl-corticosteron. Ferner kommen solche Corticoide mit den o. a. Bezeichnungen in Betracht, die anstelle einer 6α-Fluor- und/oder 9α-Fluor- und/oder 11β-Hydroxygruppe eine in entsprechender Konfiguration orientierte Chlorgruppe aufweisen. Vorzugsweise kommen von den genannten Steroid-21-alkyl- oder -arylsulfonat-17-alkylcarbonaten die 17-Methyl-, 17-Äthyl-, 17-n-Propyl-, 17-n-Butyl- und 17-n-Pentylcarbonate sowie die 21-Methan-, 21-Benzol-, 21-Toluol-, 21-p-Chlorbenzol-, 21-p-Nitrobenzolsulfonsäureester zum Einsatz. Von den genannten Steroid-17,21-dialkylorthocarbonaten werden die 17,21-Dimethyl-, 17,21-Diäthyl-, 17,21-Di-n-propyl-, 17,21-n-Butyl-, 17,21-n-Pentylorthocarbonate bevorzugt.

Der bei der ersten Verfahrensvariante zu erfolgende nukleophile Austausch der 21-Alkyl- bzw. 21-Arylsulfonsäureestergruppe gegen ein Halogenatom, wie Fluor, Chlor, Brom oder Jod, wird nach an sich bekannten Methoden durchgeführt. Hierzu werden die Steroid-21-sulfonsäure-17-alkylcarbonate mit einem Alkali-, Erdalkali- oder Trialkylammonium-fluorid-, -chlorid, -bromid oder -jodid, wobei als Alkali vorzugsweise Lithium, Natrium oder Kalium, als Erdalkali Magnesium oder Calcium und als Trialkylammonium Trimethyl-, Triäthyl-, Tripropyl-, Tributylammonium in Betracht kommen, in einem inerten, vorzugsweisen aprotischen und polaren Lösungsmittel, wie z. B. Aceton, Äthylmethylketon, Dimethylformamid, N,N-Dimethylacetamid, Acetonitril, N-Methylpyrrolidon, Hexamethylphosphorsäuretriamid, Dimethylsulfoxyd oder gegebenenfalls in Gemischen dieser Lösungsmittel unter möglichst wasserfreien Reaktionsbedingungen bei Temperaturen von 0° bis zu den Siedepunkten der verwendeten Lösungsmittel (bzw. -Gemische), vorzugsweise bei Temperaturen von 70° – 120° C, 1 Minute bis 7 Tage lang, vorzugsweise 0,5 bis 16 Stunden, umgesetzt und in üblicher Weise durch Eingießen in gegebenenfalls Kochsalz enthaltendes Wasser, Abfiltrieren des festen oder öligen Niederschlags bzw. Extrahieren mit einem geeigneten organischen Lösungsmittel, Waschen mit Wasser, gegebenenfalls Abdestillieren der Lösungsmittel und gegebenenfalls Umkristallieren aus geeigneten Lösungsmitteln (bzw. -Gemischen) isoliert bzw. gereinigt.

Eine besonders günstige und schonende Durchführungsform dieses Verfahrensschrittes des erfindungsgemäßen Verfahrens besteht darin, daß man die Kationen der obengenannten Metallhalogenide mit zur Komplexbildung geeigneten Verbindungen vorzugsweise mit »Kronenäthern« komplexiert (Kryptatbildung), wodurch wegen der dadurch bewirkten Erhöhung des Nukleophiliegrades der zuständigen Halogenidionen ein entsprechender Austausch Sulfonatrest gegen Halogen schon bei einer Temperatur von 0° bis 30° C in praktikabler und ausreichender Weise erfolgt.

Hierzu löst man in einer beispielsweisen Ausführungsform jeweils die Steroid-17-alkylcarbonat-21-sulfonsäureester in einem der oben angegebenen Lösungsmittel bzw. Lösungsmittelgemische, wobei zusätzlich chlorierte Kohlenwasserstoffe wie z. B. Chloroform, Methylenchlorid sowie nicht chlorierte Kohlenwasserstoffe wie z. B. Benzol, Toluol, Cyclohexan zur Anwendung kommen können, und versetzt diese Lösungen entweder mit 1 bis 3 Moläquivalent einer vorher bereiteten Kryptatlösung, die in bekannter Weise aus einem der oben angeführten Metallhalogenide durch Umsetzung mit einem Kronenäther (z. B. 18-Krone-6) oder einem Polyoxadiazamakrobicyclus (z. B. Kryptofix(R) 211 oder 221 oder 222) in Gegenwart einer der eben erwähnten Lösungsmittel gegebenenfalls unter Hinzufügen von katalytischen Mengen eines niedermolekularen Alkohols, vorzugsweise Methanol oder Äthanol, hergestellt wird, oder mit 1 bis 5 Moläquivalent eines der angegebenen Metallhalogenide unter Zusatz von katalytischen Mengen Kronenäther oder Polyoxadiazamakrobicyclen vom o. a. Typ (0,001 bis 0,01 Moläquivalent) läßt 24 Stunden bis 14 Tage, vorzugsweise 7 Tage bei Temperatur von 0° bis 60° C, vorzugsweise Raumtemperatur, reagieren und arbeitet wie üblich auf.

Ebenfalls eine starke Beschleunigung des nukleophilen Austausches der Steroid-21-sulfonsäureestergruppe gegen eines der o. a. Halogenidionen wird durch die sogenannte »Phasentransfer-Katalyse« erreicht, bei der die nukleophilen Austausch-Reaktionen an der Grenzfläche zwischen einem organischen Lösungsmittel bzw. Lösungsmittelgemisch und einer wäßrigen Phase in Gegenwart von katalytischen Mengen eines quartären Ammonium- oder Phosphoniumsalzes oder auch eines Kronenäthers unter Zusatz der auszutauschenden Halogenidionen als Salze abläuft.

Hierzu löst man beispielsweise das Steroid-21-sulfonat in einem der o. a. Lösungsmittel, vorzugsweise Acrylnitril oder Dimethylsulfoxyd, fügt eine Lösung eines der o. a. Alkali- oder Erdalkalihalogenide, wie z. B. Kaliumfluorid, -chlorid-, -bromid oder -jodid in Wasser sowie 0,01 bis 1 Moläquivalent eines quartären Ammoniumhalogenids bzw. -sulfonats als Katalysator, beispielsweise Tricaprylmethyl-, Tri-n-octylmethylammoniumchlorid, Tributylhexadecylphosphoniumjodid oder -bromid oder statt dessen als Katalysator einen Kronenäther, beispielsweise »[18]Krone-6«, hinzu und behandelt das Reaktionsgemisch 10 Minuten bis 100 Stunden, vorzugsweise 30 Minuten bis 48 Stunden bei Temperaturen von 0° C bis zum Siedepunkt der Reaktionsgemische, vorzugsweise bei Temperaturen von 60° bis 110° C.

Anschließend wird in üblicher Weise, beispielsweise durch Extraktion mit einem vorzugsweisen chlorierten organischen Lösungsmittel, z. B. Methylenchlorid oder Chloroform ein- oder mehrfach extrahiert, mit Wasser gewaschen, und das Lösungsmittel abdestilliert. Der Rückstand wird wie üblich zur Reinsubstanz aufgearbeitet.

In einer weiteren zweiten Verfahrensvariante können die erfindungsgemäßen Steroid-17-alkylcarbonat-21-halogenide auch aus den zugrunde liegenden Steroid-17,21-dialkylorthocarbonaten erhalten werden, indem man letztere mit Halogen abgebenden anorganischen oder organischen Säurehalogenidverbindungen, zum Beispiel mit einem Silylhalogenid, vorzugsweise Triäthyl- oder Trimethylsilylchlorid, -bromid, -jodid, -fluorid oder mit einem Phosphorig- bzw. Phosphorsäurehalogenid, vorzugsweise Phosphoroxychlorid, Phosphortri- und -pentachlorid, -bromid, oder mit einem Acylchlorid, vorzugsweise Acetyl- und Oxalylchlorid, oder mit einem Sulfonylhalogenid, vorzugsweise p-Toluolsulfonsäure- und Methansulfonsäurechlorid, oder mit einem N-Halogenamid oder -imid, vorzugsweise N-Chlorsuccinimid, N.N-Dibrombenzolsulfonamid, oder mit einem Triphenylalkylhalogenid, vorzugsweise Triphenylmethylchlorid, in einem der oben angegebenen organischen Lösungsmittel bzw. eines Lösungsmittelgemisches 1 Minute bis 60 Stunden, vorzugsweise 5 Minuten bis 24 Stunden, bei Temperaturen von 0°C bis zu den Siedepunkten der angegebenen Lösungsmittel, vorzugsweise bei Raumtemperatur bis 120°C, umsetzt. Hierbei erfolgt die Ringaufspaltung des 17,21-Dialkylorthocarbonats in der Weise, daß das Halogen die 21-Position einnimmt und das cyclische Orthocarbonat zum linearen 17-Alkylcarbonat geöffnet wird.

Die gemäß der Erfindung benötigten Steroid-17-alkylcarbonat-21-sulfonsäureester bzw. 21-ole werden gemäß der deutschen Patentanmeldung DOS 2 735 110 erhalten, während die zugrunde liegenden Steroid-17,21-dialkylorthocarbonate nach der deutschen Patentschrift DBP 1 668 079 darstellbar sind.

Die Verfahrensprodukte besitzen wertvolle pharmakologische Eigenschaften. Sie sind insbesondere lokal und topisch sehr stark antiinflammatorisch wirksam und zeigen in vorteilhafter Weise ein günstiges Verhältnis von lokaler zu systemischer antiinflammatorischer Wirkung, wie aus pharmakologischen Standardtests hergeleitet werden kann.

Aufgrund ihrer sehr starken lokalen und topischen antiphlogistischen Wirkung können die Verfahrensprodukte vorteilhaft in der Veterinär- und Humantherapie zur Behandlung von entzündlichen Dermatosen verschiedenster Genese in Form von Suspensionen, Salben, Cremes, Lösungen, Sprays usw. Verwendung finden. Als Dosierung kommen bei Behandlung von Mensch und Tier Zubereitungen mit einem Konzentrationsbereich von 0,025 – 1% in Betracht. Dabei ist als besonders vorteilhaft für die lokale und topische Therapieform herauszuheben, daß die Verfahrensprodukte aufgrund ihres günstigen Verhältnisses von lokaler zu systemischer antiphlogistischer Wirkung auch bei hochdosierter und langanhaltender Therapie praktisch nur geringfügige systemische Nebenwirkungen hervorrufen können. Darüber hinaus weisen die eingesetzten Verfahrensprodukte eine signifikant bessere Säurestabilität auf als die ihnen zugrunde liegenden cyclischen Corticoid-17,21-orthocarbonate. Dieser Tatbestand ist für eine sichere und therapiegerechte Anwendung der Produkte von ausschlaggebender Relevanz.

## Beispiele

Die Schmelzpunkte wurden im Apparat nach Tottoli (Fa. Büchi) bestimmt und sind nicht korrigiert.

Die IR-Spektren (in KBr) wurden mit dem Gitterspektrophotometer Perkin-Elmer 521 aufgenommen. Es werden jeweils nur die charakteristischen Banden angeführt. Die Aufnahme der UV-Spektren (in Methanol) erfolgte mit dem Spektralphotometer Beckman DK 1 A. Die massenspektroskopischen Untersuchungen (MS) wurden mit dem Gerät MS 9 (Fa. AEI) durchgeführt.

Für die Dünnschichtchromatographie (DC) dienten Fertigplatten Kieselgel $F_{254}$ (Fa. Merck). Wenn nicht anders angegeben, wurde als Laufmittel Methylenchlorid : Methanol = 19 : 1 benutzt. Es wurde jeweils einmal entwickelt. Die Flecken wurden durch Besprühen mit 10%iger methanolischer Schwefelsäure sowie durch Erhitzen auf 100°C sichtbar gemacht. Die $R_f$-Werte sind immer nur relativ zu verstehen.

Zur Säulenchromatographie wurde Kieselgel 60, Korngröße 0,063 – 0,2 mm (Fa. Merck) verwendet.

## Beispiel 1

a) Eine Lösung von 1 g Prednisolon-17-äthylcarbonat-21-methansulfonat in 20 ml absol. Dimethylformamid wird mit 2,2 g trockenem Lithiumchlorid versetzt und in Stickstoffatmosphäre 2 Stunden bei 100°C gerührt. Nach dem Eingießen der Reaktionslösung in etwa 150 ml Kochsalz enthaltendes Wasser wird erschöpfend mit Methylenchlorid extrahiert. Die organische Phase wird mit Wasser gewaschen, getrocknet und anschließend durch Destillation im Vakuum vom Lösungsmittel befreit. Man erhält 1,2 g eines Schaums, der an 30 g Kieselgel (Säulenmaße 2,5 x 10 cm) mit Methylenchlorid chromatographiert wird. Die Eluate, die im Dünnschichtdiagramm einen weitgehend einheitlichen $R_F$-Wert von $R_F \cong 0,60$ aufweisen, werden vereinigt und durch Abdestillieren von Lösungsmittel befreit. Der Rückstand wird aus Methanol/Diisopropyläther umkristallisiert und ergibt 690 mg 21-Desoxy-prednisolon-17-äthylcarbonat-21-chlorid vom Schmp. 167 – 169°C.

Charakt. IR-Banden: 3520, 3430, 1740, 1725, 1650, 1270 $cm^{-1}$
Massenspektrum: $M^{\oplus}=450$
DC: $R_F = 0,60$
UV: $\lambda max. = 238$ nm; $\varepsilon = 15100$
Beilsteinprobe: positiv

b) Zum gleichen Verfahrensprodukt mit den gleichen Daten, wie unter Beispiel 1 a) angegeben, gelangt man, wenn man anstelle des Prednisolons-17-äthylcarbonat-21-methansulfonats eine äquimolekulare Menge von Prednisolon-17-äthylcarbonat-21-toluolsulfonat oder -21-p-Chlorbenzolsulfonat in die Reaktion einsetzt und in gleicher Weise, wie unter Beispiel 1 a) angegeben, umsetzt und aufarbeitet.

Beispiel 2

Wird in die Reaktion gemäß Beispiel 1 a) oder 1 b) anstelle des Lithiumchlorids eine äquimolekulare Menge Lithiumbromid eingesetzt und in gleicher Weise, wie dort angegeben, umgesetzt und aufgearbeitet, so erhält man nach dem Kristallisieren aus Methanol/Äther 490 mg 21-Desoxy-prednisolon-17-äthylcarbonat-21-bromid vom Schmp. 164—166° C.

Charakt. IR-Banden: 3440, 1730, 1655, 1610, 1270 $cm^{-1}$
DC: $R_F = 0,63$
UV: $\lambda max. = 238$ nm; $\varepsilon = 14750$
Beilsteinprobe: positiv

Beispiel 3

Eine Lösung von 2,3 g Prednisolon-17-äthylcarbonat-21-p-chlorbenzolsulfonat in 46 ml absol. Äthylmethylketon wird mit 460 mg Natriumjodid versetzt. Nach 2stündigem Rückflußkochen (nach etwa 30 Minuten wird hier eine Ausfällung beobachtet) wird in 450 ml Kochsalz enthaltendes Wasser eingerührt und erschöpfend mit Methylenchlorid extrahiert. Nach dem Waschen der vereinigten Extrakte mit Wasser, Trocknen und Abdestillieren des Lösungsmittels erhält man einen schaumigen Rückstand, der mit Diäthyläther zur »Kristallisation« gebracht wird. Man erhält 2,1 g amorphes 21-Desoxy-prednisolon-17-äthylcarbonat-21-jodid.

Charakt. IR-Banden: 3400, 1730, 1650, 1610, 1270 $cm^{-1}$
UV: $\lambda max. = 238$ nm; $\varepsilon = 15150$
DC: $R_F = 0,50$ (Laufmittel = Toluol : Methanol : Aceton = 80 : 20 : 5)
Analyse: Jod: Ber.: 23,4% Gef.: 21,6%

Beispiel 4

21-Desoxy-21-fluor-prednisolon-17-äthylcarbonat

1 g Prednisolon-17-äthylcarbonat-21-methansulfonat werden zu einer vorher bereits 35 Minuten bei Raumtemperatur gerührten Suspension von 145 mg 18-Krone-6 und 190 mg Kaliumfluorid in 8 ml Acetonitril gegeben. Dann wird in einer $N_2$-Atmosphäre 8 Stunden lang bei 80° C gerührt. Nach dem Abkühlen wird das Reaktionsgemisch in 50 ml Wasser eingerührt und wie üblich (Extrahieren mit Methylenchlorid, mehrmaliges Waschen mit Wasser, Abdestillieren der Lösungsmittel im Vakuum, chromatographische Reinigung wie in Beispiel 1 beschrieben, Umkristallisieren des DC-einheitlichen Eluat-Rückstands aus Methanol/Diisopropyläther) aufgearbeitet. Es werden 310 mg 21-Desoxy-21-fluor-prednisolon-17-äthylcarbonat erhalten.

Schmp. 170° C
IR: 3520, 1735, 1720, 1650, 1270 $cm^{-1}$
UV: $\lambda max. = 238$ nm, $\varepsilon = 1480$

Herstellung von Prednisolon-17-äthylcarbonat-21-p-chlorbenzolsulfonat

Zu einer Lösung von 3 g Prednisolon-17-äthylcarbonat in 35 ml abs. Aceton und 12 ml abs. Pyridin werden bei 0° C 1,8 g p-Chlorbenzolsulfonsäurechlorid zugegeben. Nach 20 Stunden Rühren bei 0° bis 22° C (allmählich Temperatur ansteigen lassen) wird in Wasser eingegossen (das ausgefallene Öl

abfiltriert und dieses mit Methylenchlorid aufgenommen, dann wird gewaschen und das Lösungsmittel im Vakuum eingeengt. Der Rückstand wird an Kieselgel (Säule 4 × 14 cm) mit Methylenchlorid als Eluationsmittel chromatographiert. Die DC-reinen Fraktionen mit $R_f$ = 0,60 werden vereinigt und aus Äthanol/Äther kristallisiert. Man erhält 2,6 g Prednisolon-17-äthylcarbonat-21-p-chlorbenzolsulfonat vom Schmp. 188° C.

Massenspektrum: $M^+$ = 605
IR: 3430, 3320, 1740, 1655, 1620, 1600, 1510, 1370, 1280, 1190, 1030 $cm^{-1}$

## Beispiel 5

a) Eine Lösung von 1 g Prednisolon-17-n-propylcarbonat-21-methansulfonat in 20 ml absol. Dimethylformamid wird mit 2,2 g trockenem Lithiumchlorid versetzt und in Stickstoffatmosphäre 2 Stunden bei 100° C gerührt. Nach dem Eingießen der Reaktionslösung in etwa 150 ml Kochsalz enthaltendes Wasser wird erschöpfend mit Methylenchlorid extrahiert. Die organische Phase wird mit Wasser gewaschen, getrocknet und anschließend durch Destillation im Vakuum vom Lösungsmittel befreit. Man erhält 1,2 g eines Schaums, der an 30 g Kieselgel (Säulenmaße 2,5 × 10 cm) mit Methylenchlorid chromatographiert wird. Die Eluate, die im Dünnschichtdiagramm einen weitgehend einheitlichen $R_F$-Wert von $R_F$ = 0,60 aufweisen, werden vereinigt und durch Abdestillieren vom Lösungsmittel befreit. Der Rückstand wird aus Methanol/Diisopropyläther umkristallisiert und ergibt 710 mg 21-Desoxyprednisolon-17-n-propylcarbonat-21-chlorid vom Schmp. 128° – 130° C.

Charakt. IR-Banden: 3420, 1730, 1650, 1610, 1270 – 1280 $cm^{-1}$
Massenspektrum: $M^{\oplus}$ = 464
DC: $R_F$ = 0,60
UV: $\lambda max.$ = 238 nm; $\varepsilon$ = 15300

b) Zum gleichen Verfahrensprodukt mit den gleichen Daten, wie unter Beispiel 5 a) angegeben, gelangt man, wenn man anstelle des Prednisolons-17-n-propylcarbonat-21-methansulfonats eine äquimolekulare Menge von Prednisolon-17-n-propylcarbonat-21-toluolsulfonat oder -21-p-chlorbenzolsulfonat in die Reaktion einsetzt und in gleicher Weise, wie unter Beispiel 5. a) angegeben, umsetzt und aufarbeitet.

## Beispiel 6

Wird in die Reaktion gemäß Beispiel 5 a) oder 5 b) anstelle des Lithiumchlorids eine äquimolekulare Menge Lithiumbromid eingesetzt und in gleicher Weise, wie dort angegeben, umgesetzt und aufgearbeitet, so erhält man nach dem Kristallisieren aus Methanol/Äther 490 mg 21-Desoxy-prednisolon-17-n-propylcarbonat-21-bromid vom Schmp. 102 – 105° C.

Charakt. IR-Banden: 3440, 1730, 1650, 1610, 1270 $cm^{-1}$
DC: $R_F$ = 0,63
UV: $\lambda max.$ = 238 nm; $\varepsilon$ = 14900

## Beispiel 7

Eine Lösung von 2,3 g Prednisolon-17-n-propylcarbonat-21-p-chlorbenzolsulfonat in 46 ml absol. Äthylmethylketon wird mit 460 mg Natriumjodid versetzt. Nach 2stündigem Rückflußkochen wird in 450 ml Kochsalz enthaltendes Wasser eingerührt und erschöpfend mit Methylenchlorid extrahiert. Nach dem Waschen der vereinigten Extrakte mit Wasser, Trocknen und Abdestillieren des Lösungsmittels, erhält man 2 g eines schaumigen Rückstandes, der das 21-Desoxy-prednisolon-17-n-propylcarbonat-21-jodid darstellt.

Charakt. IR-Banden: 3420, 1730, 1650, 1610, 1255 $cm^{-1}$
Beilsteinprobe: positiv
DC: $R_F$ = 0,50 (Laufmittel = Toluol : Methanol : Aceton = 80 : 20 : 5)
UV: $\lambda max.$ = 237 nm; $\varepsilon$ = 13800

Beispiel 8

In gleicher Weise, wie in Beispiel 4 beschrieben, werden 1 g Prednisolon-17-n-propylcarbonat-21-p-chlorbenzolsulfonat umgesetzt und aufgearbeitet. Man erhält das 21-Desoxy-prednisolon-17-n-propylcarbonat-21-fluorid.

UV: λmax. = 238 nm; ε = 13900

Herstellung von Prednisolon-17-n-propylcarbonat-21-p-chlorbenzol-sulfonat

Zu einer Lösung von 3 g Prednisolon-17-n-propylcarbonat in 35 ml abs. Aceton und 12 ml abs. Pyridin werden bei 0° C 1,75 g p-Chlorbenzolsulfonsäurechlorid zugetropft. Nach 20 Stunden Rühren bei 0° bis 22° C (allmählich Temperatur ansteigen lassen) wird in Wasser eingegossen, mit Methylenchlorid extrahiert, gewaschen und das Extraktionsmittel im Vakuum eingeengt. Der Rückstand wird an Kieselgel (Säule 4 × 14 cm) mit Methylenchlorid als Eluationsmittel chromatographiert. Die DC-reinen Fraktionen mit $R_f$ = 0,60 werden vereinigt und aus Äthanol/Äther kristallisiert.
Man erhält 2,5 g Prednisolon-17-n-propylcarbonat-21-p-chlorbenzolsulfonat vom Schmp. 105° C.

Massenspektrum: $M^{\oplus}$ = 620
IR: 3430, 1730, 1655, 1610, 1600, 1350, 1265, 1170, 1030 $cm^{-1}$

Beispiel 9

In gleicher Weise, wie in Beispiel 1 a) beschrieben werden 1 g Prednisolon-17-n-butylcarbonat-21-methansulfonat mit 2 g Lithiumchlorid umgesetzt und aufgearbeitet. Man erhält nach dem Kristallisieren aus Diisopropyläther (Anreiben) 670 mg 21-Desoxy-prednisolon-17-n-butylcarbonat-21-chlorid vom Schmp. 151 – 155° C.

Charakt. IR-Banden: 3430, 1740, 1660, 1610, 1270 $cm^{-1}$
UV: λmax. = 238 nm; ε = 14750
Beilsteinprobe positiv

Beispiel 10

In gleicher Weise, wie in Beispiel 1 a) beschrieben werden 1 g Prednisolon-17-n-butylcarbonat-21-methansulfonat mit 4,5 g Lithiumbromid umgesetzt und aufgearbeitet. Man erhält nach dem Anspritzen mit Diisopropyläther 685 mg 21-Desoxy-prednisolon-17-n-butylcarbonat-21-bromid vom Schmp. ca. 140° C (Zers.).

UV: λmax. = 238 nm; ε = 15200
Beilsteinprobe positiv

Beispiel 11

In gleicher Weise, wie in Beispiel 3 beschrieben werden 2,3 g Prednisolon-17-n-butylcarbonat-21-p-chlorbenzolsulfonat mit 460 mg Natriumjodid umgesetzt und aufgearbeitet. Man erhält nach dem Anspritzen mit Äther 2,3 g 21-Desoxy-prednisolon-17-n-butylcarbonat-21-jodid. Schmp. ca. 100° C (Zers.).

UV: λmax. = 237 nm; ε = 13600
Beilsteinprobe positiv

In gleicher Weise, wie in Beispiel 4 beschrieben, werden 1 g Prednisolon-17-n-butylcarbonat-21-p-chlorbenzolsulfonat umgesetzt und aufgearbeitet. Man erhält das 21-Desoxy-prednisolon-17-n-butylcarbonat-21-fluorid.

UV: λmax. = 238 nm; ε = 14400

Prednisolon-17-n-butylcarbonat-21-p-chlorbenzolsulfonat wird in gleicher Weise aus Prednisolon-17-n-butylcarbonat und p-Chlorbenzolsulfänsäurechlorid hergestellt, wie im Anschluß an Beispiel 4 für die Darstellung von Prednisolon-17-äthylcarbonat-21-p-chlorbenzolsulfonat beschrieben worden ist.

## Beispiel 12

In gleicher Weise, wie in Beispiel 4 beschrieben, werden 1 g Prednisolon-17-n-butylcarbonat-21-p-chlorbenzolsulfonat umgesetzt und aufgearbeitet. Man erhält das 21-Desoxy-prednisolon-17-n-butylcarbonat-21-fluorid.

UV: λmax. = 238 nm; ε = 14400

Prednisolon-17-n-butylcarbonat-21-p-chlorbenzolsulfonat wird in gleicher Weise aus Prednisolon-17-n-butylcarbonat und p-Chlorbenzolsulfonsäurechlorid hergestellt, wie im Anschluß an Beispiel 4 für die Darstellung von Prednisolon-17-äthylcarbonat-21-p-chlorbenzolsulfonat beschrieben worden ist.

## Beispiel 13

In gleicher Weise, wie in Beispiel 1 a) beschrieben werden 1 g Prednisolon-17-n-pentylcarbonat-21-methansulfonat mit 2 g Lithiumchlorid umgesetzt und aufgearbeitet. Man erhält nach dem Kristallisieren aus Diisopropyläther (Anreiben) 540 mg 21-Desoxy-prednisolon-17-n-pentylcarbonat-21-chlorid vom Schmp. 118–124° C.

Charakt. IR-Banden: 3430, 1740, 1660, 1610, 1270 $cm^{-1}$
UV: λmax. = 238 nm; ε = 14400
Beilsteinprobe positiv

## Beispiel 14

In gleicher Weise, wie in Beispiel 1 a) beschrieben werden 1 g Prednisolon-17-n-pentylcarbonat-21-methansulfonat mit 4,5 g Lithiumbromid umgesetzt und aufgearbeitet. Man erhält nach dem Anspritzen mit Diisopropyläther 525 mg 21-Desoxy-prednisolon-17-n-pentylcarbonat-21-bromid vom Schmp. von etwa 100° C (Zers.).

UV: λmax. = 238 nm; ε = 14800
Beilsteinprobe positiv

## Beispiel 15

In gleicher Weise, wie in Beispiel 3 beschrieben, werden 2,3 g Prednisolon-17-n-pentylcarbonat-21-p-chlorbenzolsulfonat mit 460 mg Natriumjodid umgesetzt und aufgearbeitet. Man erhält nach dem Digerieren mit Äther 2,0 g amorphes 21-Desoxy-prednisolon-17-n-pentylcarbonat-21-jodid.

UV: λmax. = 238 nm; ε = 13000
Beilsteinprobe positiv

## Beispiel 16

In gleicher Weise, wie in Beispiel 4 beschrieben, werden 1 g Prednisolon-17-n-pentylcarbonat-21-p-chlorbenzolsulfonat umgesetzt und aufgearbeitet. Man erhält das 21-Desoxy-prednisolon-17-n-pentylcarbonat-21-fluorid.

UV: λmax. = 238 nm; ε = 14100

Prednisolon-17-n-pentylcarbonat-21-p-chlorbenzolsulfonat wird in gleicher Weise aus Prednisolon-17-n-pentylcarbonat und p-Chlorbenzolsulfonsäurechlorid hergestellt, wie im Anschluß an Beispiel 4 für die Darstellung von Prednisolon-17-äthylcarbonat-21-p-chlorbenzolsulfonat beschrieben worden ist.

## Beispiel 17

a) Eine Lösung von 1 g Dexamethason-17-äthylcarbonat-21-p-chlorbenzol-sulfonat in 20 ml absol. Dimethylformamid wird mit 2,2 g trockenem Lithiumchlorid versetzt und in Stickstoffatmosphäre 2 Stunden bei 100° C gerührt. Nach dem Eingießen der Reaktionslösung in etwa 100 ml Kochsalz

enthaltendes Wasser wird erschöpfend mit Methylenchlorid extrahiert. Die organische Phase wird mit Wasser gewaschen, getrocknet und anschließend durch Destillation im Vakuum vom Lösungsmittel befreit. Man erhält 1,0 g eines Schaums, der an 30 g Kieselgel (Säulenmaße 2,5 x 10 cm) mit Methylenchlorid chromatographiert wird. Die Eluate, die im Dünnschichtdiagramm einen weitgehend einheitlichen $R_F$-Wert von $R_F \cong 0,60$ aufweisen, werden vereinigt und durch Abdestillieren vom Lösungsmittel befreit. Der Rückstand wird aus Methanol/Diisopropyläther umkristallisiert und ergibt 620 mg 21-Desoxy-dexamethason-17-äthylcarbonat-21-chlorid vom Schmp. 222° C.
Wird nicht umkristallisiert, sondern nur mit Diisopropyläther digeriert, so beträgt in der Regel der Schmp. 140 – 145° C.

Charakt. IR-Banden: 3420, 1730, 1655, 1600, 1260 $cm^{-1}$
Massenspektrum: $M^{\oplus} = 482$
DC: $R_F = 0,35$ (Essigester/Toluol = 1 : 1)
UV: $\lambda max. = 238$ nm; $\varepsilon = 15000$

b) Zum gleichen Verfahrensprodukt mit den gleichen Daten, wie unter Beispiel 18 a) angegeben, gelangt man, wenn man anstelle des Dexamethason-17-äthylcarbonat-21-p-chlorbenzolsulfonats eine äquimolekulare Menge vom Dexamethason-17-äthylcarbonat-21-toluolsulfonat oder -21-methansulfonat in die Reaktion einsetzt und in gleicher Weise, wie unter Beispiel 18 a) angegeben, umsetzt und aufarbeitet.

## Beispiel 18

Wird in die Reaktion gemäß Beispiel 18 a) oder 18 b) anstelle des Lithiumchlorids eine äquimolekulare Menge Lithiumbromid eingesetzt und in gleicher Weise, wie dort angegeben, umgesetzt (statt 2 h Reaktionsdauer hier aber 3,5 h Reaktionszeit) so erhält man nach dem Eingießen des Reaktionsansatzes in Wasser, Abfiltrieren des Niederschlags, Waschen mit Wasser und Trocknen des Niederschlags über Phosphorpentoxyd im Hochvakuum bei 70° C 710 mg 21-Desoxy-dexamethason-17-äthylcarbonat-21-bromid vom Schmp. 165° C.

IR-Banden: 3430, 1725, 1655, 1610, 1270 $cm^{-1}$
DC: $R_F = 0,32$ (Toluol/Essigester = 55 : 45)
MS-Spektrum: $M^{\oplus} = 416$
Beilsteinprobe positiv
UV: $\lambda max. = 238$ nm; $\varepsilon = 15400$

## Beispiel 19

Eine Lösung von 2,3 g Dexamethason-17-äthylcarbonat-21-p-chlorbenzolsulfonat in 46 ml absol. Äthylmethylketon wird mit 800 mg Natriumjodid versetzt. Nach 2stündigem Rückflußkochen wird in 450 ml Kochsalz enthaltendes Wasser eingerührt, wobei ein Niederschlag anfällt, der abfiltriert wird. Nach dem Waschen mit Wasser, Trocknen im Vakuum über $P_2O_5$, wird der Niederschlag mit Diäthyläther digeriert. Man erhält 2,1 g 21-Desoxy-dexamethason-17-äthylcarbonat-21-jodid.

Charakt. JR-Banden: 3420, 1730, 1660, 1610, 1270 $cm^{-1}$
DC: $R_F = 0,50$ (Laufmittel = Toluol : Methanol : Aceton = 80 : 20 : 5)
Beilsteinprobe positiv
UV: $\lambda max. = 237$ nm; $\varepsilon = 14600$

## Beispiel 20

In gleicher Weise, wie in Beispiel 4 beschrieben, werden 1 g Dexamethason-17-äthylcarbonat-21-p-chlorbenzolsulfonat umgesetzt und aufgearbeitet. Man erhält das 21-Desoxy-dexamethason-17-äthylcarbonat-21-fluorid.

UV: $\lambda max. = 238$ nm; $\varepsilon = 14600$

Herstellung von Dexamethason-17-äthylcarbonat-21-p-chlorbenzolfulfonat

Zu einer Lösung von 3 g Dexamethason-17-äthylcarbonat in 35 ml abs. Aceton und 12 ml abs. Pyridin werden bei 0° C 1,8 g p-Chlorbenzolsulfonsäurechlorid zugegeben. Nach 20 Stunden Rühren bei 0° bis 22° C (allmählich Temperatur ansteigen lassen) wird in Wasser eingegossen, das ausgefallene Öl abfiltriert und dieses mit Methylenchlorid aufgenommen; dann wird gewaschen und das Lösungsmittel im Vakuum eingeengt. Der Rückstand wird mit Diisopropyläther verrieben und ergibt 3,8 g Dexamethason-17-äthylcarbonat-21-p-chlorbenzolsulfonat vom Schmp. 123° C (unter Aufschäumen).

| | |
|---|---|
| Massenspektrum: | $M^{\oplus} = 638$ |
| IR: | 3430, 1730, 1655, 1620, 1600, 1370, 1260, 1180 $cm^{-1}$ |
| UV: | $\lambda max. = 232$ nm; $\varepsilon = 24500$ |

## Beispiel 21

21-Desoxy-21-chlor-dexamethason-17-propylcarbonat

a) Dexamethason-17-propylcarbonat-21-methansulfonat
46,8 g Dexamethason-17-propylcarbonat werden in einer Mischung aus 470 ml Aceton und 173 ml Pyridin gelöst. Dann werden nach Abkühlen auf 0° C 33 ml Mesylchlorid unter Rühren eingetropft. Nach 5½stündigem Rühren bei 0–5° C wird das Reaktionsgemisch in ca. 4 l Wasser gegossen. Nach einigem Stehen wird der ausgefallene Niederschlag abgesaugt und in Methylenchlorid aufgenommen. Nach dem Waschen mit Wasser und Trocknen über Natriumsulfat wird der Methylenchloridextrakt im Vakuum zur Trockne eingedampft. Der Destillationsrückstand wird aus Toluol/Diisopropyläther umkristallisiert. Es werden so 49,3 g Dexamethason-17-propylcarbonat-21-methansulfonat vom Schmp. 161–163° C erhalten.

b) 40,0 g Dexamethason-17-propylcarbonat-21-methansulfonat werden zu einer Mischung aus 400 ml absolutem Dimethylformamid und 80,0 g Lithiumchlorid, das im Vakuum bei 120° C getrocknet worden war, gegeben. Anschließend wird das Reaktionsgemisch unter Stickstoff 6 Stunden lang bei 100° C Innentemperatur gerührt. Anschließend wird im Vakuum bei ca. 50° C Außentemperatur zur Trockne eingedampft. Es wird mit einer Mischung aus 100 ml Toluol und 100 ml Essigester verrührt und zur Entfernung der Lithiumsalze mit 200 ml Wasser ausgerührt. Nach Abtrennen der wäßrigen Phase und dem Trocknen wird abermals eingeengt und durch Digerieren mit Toluol in Essigester von schwerlöslichen Anteilen abgetrennt. Die Mutterlaugen werden einrotiert und an einer Säule aus 1,6 kg Kieselgel mit Toluol/Essigestern 4 : 1 als Laufmittel chromatographiert. Die Chromatographie wird mittels DC Laufmittel Toluol/Essigester 65 : 35 kontrolliert unter Zuhilfenahme von authentischem Material. Es werden so, nach dem Umkristallisieren aus Essigester und anschließend aus Äthanol 10,2 g 21-Desoxy-dexamethason-17-propylcarbonat-21-chlorid vom Schmp. 189–190° C erhalten.

## Beispiel 22

21-Desoxy-urbason-17-propylcarbonat-21-chlorid

1,1 g Urbason-17-propylcarbonat werden in 4 ml absolutem Piperidin gelöst und bei 0° C unter Rühren mit 1 ml Mesylchlorid versetzt. Anschließend wird noch eine Stunde lang bei Raumtemperatur nachgerührt. Dann wird das Reaktionsgemisch in ca. 50 ml eiskalter 1 n Salzsäure eingerührt.

Der ausgefallene Niederschlag wird abgesaugt, mit Wasser gewaschen und nach dem Trocknen im Vakuum über Calciumchlorid bei 30–40° C aus Methanol und wenig Äther umkristallisiert. Es werden 1,21 g Urbason-17-propylcarbonat-21-methansulfonat vom Schmp. 143–145° C erhalten.

1,0 g Urbason-17-propylcarbonat-21-mesylat wurden in 20 ml absolutem Dimethylformamid gelöst, mit 2 g wasserfreiem Lithiumchlorid versetzt und 4 Stunden lang bei 95° C Innentemperatur in einer Stickstoffatmosphäre gerührt. Danach wird das Reaktionsgemisch in 100 ml kaltes Wasser eingerührt, der resultierende Niederschlag abgesaugt, mit etwas Wasser gewaschen, der Filterrückstand in Methylenchlorid aufgenommen, abermals mit Wasser gewaschen, über Natriumsulfat getrocknet, auf ein kleines Volumen im Vakuum eingedampft und an einer Säule aus 50 g Kieselgel mittels Toluol/Essigester (3 : 1) als Laufmittel chromatographiert. Ähnlich wie in Beispiel 1 b) beschrieben, werden so nach dem Umkristallisieren aus Methylenchlorid/Hexan 380 mg 21-Desoxy-urbason-17-propylcarbonat-21-chlorid vom Schmp. 117–120° C erhalten.

## Beispiel 23

### 16α-Methyl-21-desoxy-prednisolon-17-n-propylcarbonat-9α,21-dichlorid

0,544 g 9α-Chlor-16α-methyl-prednisolon-17-n-propylcarbonat wurden in 2 ml absolutem Pyridin gelöst, anschließend bei 0° C mit 0,5 ml Methansulfonsäurechlorid versetzt. Danach wird noch 30 Minuten bei Raumtemperatur gerührt und anschließend wird das Reaktionsgemisch in 25 ml eiskalter 1 n Salzsäure eingerührt. Der ausgefallene Niederschlag wird abgesaugt, mit Wasser gewaschen und aus Methanol umkristallisiert. Es werden 470 mg 9α-Chlor-16α-methyl-prednisolon-17-n-propylcarbonat-21-methansulfonat vom Schmp. 203–204° C erhalten. 200 m dieses 21-Methansulfonates werden in 4 ml absolutem Dimethylformamid gelöst. Nach Zugabe von 400 mg trockenem Lithiumbromid wird 6 Stunden lang unter Rühren und in einer Stickstoffatmosphäre auf 100° C erwärmt. Anschließend wird in 25 ml Wasser eingerührt, der ausgefallene Niederschlag abgesaugt, in Methylenchlorid aufgenommen und mit etwas Wasser gewaschen. Nach dem Trocknen über Natriumsulfat wird im Vakuum zur Trockne eingedampft und der Destillationsrückstand in sehr wenig Methylenchlorid gelöst, auf eine Säule aus 20 g Kieselgel aufgetragen und mit Methylenchlorid als Laufmittel chromatographiert (wie in Beispiel 22 beschrieben mittels DC kontrolliert). Die authentischen Fraktionen wurden gesammelt, im Vakuum eingeengt.

Nach dem Umkristallisieren aus Diisopropyläther/Petroläther werden 115 mg 16α-Methyl-21-desoxy-prednisolon-17-n-propylcarbonat-9α,21-dichlorid vom Schmp. 193–196° C erhalten.

Das als Ausgangsmaterial verwendete 9α-Chlor-16α-methyl-prednisolon-17-n-propylcarbonat kann in Anlehnung an die eigene Anmeldung DOS 1 668 079 und vorteilhaft wie folgt erhalten werden:

3,3 g 9α-Chlor-16α-methyl-prednisolon wurden in 97 ml absolutem Dioxan gelöst und nach Zugabe von 3,5 g Tetrapropylorthocarbonat und 190 mg p-Toluolsulfonsäure 4 Stunden lang bei Raumtemperatur gerührt. Anschließend wird in ca. 500 ml ca. 1%ige wäßrige Natriumhydrogencarbonatlösung eingerührt. Der ausgefallene Niederschlag wird abgesaugt und aus Äthanol/Petroläther umkristallisiert. Es wird in wenig Methylenchlorid, das 1% Methanol enthält, gelöst und an 20 g Kieselgel mit demselben Lösungsmittelgemisch als Elutionsmittel chromatographiert. Es werden so, ebenfalls wieder mittels Kontrolle der einzelnen Fraktionen mittels DC, 2,68 g 9α-Chlor-16α-methyl-prednisolon-17,21-dipropylorthocarbonat vom Schmp. 222–225° C erhalten. 2,5 g dieses Orthocarbonats werden zu 136 ml Eisessig, das 0,61 ml Wasser enthält, gegeben und 4 Stunden bei Raumtemperatur gerührt. Nach dem Einrühren in 500 ml Kochsalzlösung wird der ausgefallene Niederschlag abgesaugt, mit etwas Wasser gewaschen und bei 80° C im Hochvakuum getrocknet. Es werden 2,2 g 9α-Chlor-16α-methyl-prednisolon-17-n-propylcarbonat vom Schmp. 230–235° C erhalten, das für die weiteren Umsetzungen von ausreichender Reinheit ist.

## Beispiel 24

### 6α-Methyl-21-desoxy-21-chlor-prednisolon-17-propylcarbonat

1 g Urbason-17-propylcarbonat-21-methansulfonat werden in 27 ml Aceton gelöst. Dazu werden bei 0° C und in einer $N_2$-Atmosphäre langsam unter Rühren 0,7 ml einer $CrO_3$-Lösung nach Jones (hergestellt durch Auflösen von 6,67 g Chormtrioxyd in einer Mischung aus 25 ml Wasser und 5,33 ml konzentrierter Schwefelsäure) zugetropft.

Anschließend wird 2 Stunden lang bei 0° C und noch 1 Stunde bei Raumtemperatur nachgerührt. Anschließend wird mit einer Mischung aus Diisopropyläther und Essigester extrahiert und die organische Phase gut mit Wasser gewaschen und über Natriumsulfat getrocknet. Nach dem Einengen im Vakuum wird das resultierende Öl mit Diisopropyläther zur Kristallisation gebracht. Es werden so 0,78 g 6α-Methyl-prednisolon-17-propylcarbonat-21-methansulfonat vom Schmp. 92–95° C erhalten.

Davon wurden 700 mg zu einer Mischung aus 7 ml absolutem Dimethylsulformamid und 1,4 g trockenem Lithiumchlorid unter strengem Feuchtigkeitsausschluß gegeben und 4 Stunden lang auf 90° C in einer trockenen Stickstoffatmosphäre unter Rühren erwärmt. Im übrigen wird, wie im Beispiel 1 beschrieben, aufgearbeitet (Chromatographie an 30 g Kieselgel). Es wurde 175 mg 6α-Methyl-21-desoxy-21-chlor-prednison-17-propylcarbonat vom Schmp. 123–126° C erhalten.

## Beispiel 25

### 9α-Fluor-16α-methyl-21-desoxy-21-chlor-prednison-17-propylcarbonat

2,7 g Dexamethason-17-propylcarbonat-21-methansulfonat, gelöst in 81 ml Aceton (s. Beispiel 9), wurden, wie unter Beispiel 4 beschrieben, mit 2,16 ml Reagenz nach Jones behandelt. Nach der üblichen Aufarbeitung werden 2,27 g 9α-Fluor-16α-methyl-prednison-17-propylcarbonat-21-methansulfonat vom Schmp. 100–105° C (Zers.) erhalten.

Hiervon werden 1,5 g in einer Mischung aus 55 ml Aceton (wasserfrei) und 34 ml trockenem Dimethylformamid gelöst und nach Zugabe von 3 g trockenem Lithiumchlorid 6 Stungen lang unter Rückfluß und Rühren gekocht. Anschließend wird unter Stickstoff im Vakuum auf ca. 1/3 des Volumens eingeengt und dann, wie in Beispiel 3 beschrieben, aufgearbeitet.

Es werden 840 mg 9α-Fluor-16α-methyl-21-desoxy-21-chlor-prednison-17-propylcarbonat vom Schmp. 152—155° C erhalten.

## Beispiel 26

In gleicher Weise wie in Beispiel 18 a) beschrieben, werden 1 g Dexamethason-17-n-butylcarbonat-21-p-chlorbenzolsulfonat mit 2 g Lithiumchlorid umgesetzt und aufgearbeitet. Man erhält nach dem Kristallisieren aus Diisopropyläther (Anreiben) 600 mg 21-Desoxy-dexamethason-17-n-butylcarbonat-21-chlorid vom Schmp. 136° C.

Charakt. IR-Banden: 3430, 1730, 1660, 1610, 1260—1280 $cm^{-1}$
UV: λmax. = 238 nm; ε = 15200
MS: $M^{\oplus}$ = 510

## Beispiel 27

In gleicher Weise wie in Beispiel 18 a) beschrieben, werden 1 g Dexamethason-17-n-butylcarbonat-21-methansulfonat mit 4,5 g Lithiumbromid umgesetzt und aufgearbeitet. Man erhält nach dem Anspritzen mit Diisopropyläther 585 mg 21-Desoxy-dexamethason-17-n-butylcarbonat-21-bromid vom Schmp. 120—125° C (Zers.).

UV: λmax. = 238 nm; ε = 14750

## Beispiel 28

In gleicher Weise wie in Beispiel 20 beschrieben, werden 2,3 g Dexamethason-17-n-butylcarbonat-21-p-chlorbenzolsulfonat mit 460 mg Natriumjodid umgesetzt und aufgearbeitet. Man erhält nach dem Anspritzen mit Äther 2,0 g 21-Desoxy-dexamethason-17-n-butylcarbonat-21-jodid.

UV: λmax. = 238 nm; ε = 13600
Beilsteinprobe positiv

Dexamethason-17-n-butylcarbonat-21-p-chlorbenzolsulfonat wird in gleicher Weise aus Dexamethason-17-n-butylcarbonat und p-chlorbenzolsulfonsäurechlorid hergestellt, wie im Anschluß an Beispiel 4 für die Darstellung von Prednisolon-17-äthylcarbonat-21-p-chlorbenzolsulfonat beschrieben worden ist.

IR: 3430, 1730, 1660, 1610, 1600, 1370, 1270, 1180 $cm^{-1}$
UV: λmax. = 231 nm; ε = 23700

## Beispiel 29

In gleicher Weise wie in Beispiel 18 a) beschrieben, werden 1 g Dexamethason-17-n-pentylcarbonat-21-methansulfonat mit 2 g Lithiumchlorid umgesetzt und aufgearbeitet. Man erhält nach dem Kristallieren aus Diisopropyläther + Cyclohexan (Anreiben) 480 mg amorphes 21-Desoxy-dexamethason-17-n-pentylcarbonat-21-chlorid.

Charakt. IR-Banden: 3430, 1740, 1660, 1610, 1270 $cm^{-1}$
UV: λmax. = 238 nm; ε = 13600
Beilsteinprobe positiv

## Beispiel 30

In gleicher Weise wie in Beispiel 18 a) beschrieben, werden 1 g Dexamethason-17-n-pentylcarbonat-21-methansulfonat mit 4,5 g Lithiumbromid umgesetzt und aufgearbeitet. Man erhält nach dem

Anspritzen mit Diisopropyläther/Hexan 520 mg amorphes 21-Desoxy-dexamethason-17-n-pentylcarbonat-21-bromid.

UV: λmax. = 238 n,; ε = 13400

Beilsteinprobe positiv

Beispiel 31

In gleicher Weise wie in Beispiel 20 beschrieben, werden 2,3 g Dexamethason-17-n-pentylcarbonat-21-p-chlorbenzolsulfonat mit 460 mg Natriumjodid umgesetzt und aufgearbeitet. Man erhält nach dem Digerieren mit Diisopropyläther 2,0 g amorphes 21-Desoxy-dexamethason-17-n-pentylcarbonat-21-jodid.

UV: λmax. = 238 nm; ε = 12800
Beilsteinprobe positiv

Dexamethason-17-n-pentylcarbonat-21-p-chlorbenzolsulfonat wird in gleicher Weise aus Dexamethason-17-n-pentylcarbonat und p-chlorbenzolsulfonsäurechlorid hergestellt, wie im Anschluß an Beispiel 4 für die Darstellung von Prednisolon-17-äthylcarbonat-21-p-chlorbenzolsulfonat beschrieben worden ist.

Beispiel 32

a) Eine Lösung von 10 g Dexamethason-17-n-propylcarbonat-21-p-chlorbenzolsulfonat in 200 ml absol. Dimethylformamid wird mit 22 g trockenem Lithiumchlorid versetzt und in Stickstoffatmosphäre 1/2 bis 1 Std. bei 100°C gerührt. Nach dem Eingießen der Reaktionslösung in etwa 1 l Kochsalz enthaltendes Wasser erhält man einen Niederschlag, der abfiltriert, mit Wasser gewaschen und über $P_2O_5$ im Vakuum getrocknet wird. Der erhaltene trockene Niederschlag (7,4 g) wird an 500 g Kieselgel (Säulenmaße 4,5×70 cm) mit Toluol/Essigester ≅ 4 : 1 chromatographiert. Die Eluate, die im Dünnschichtdiagramm einen weitgehend einheitlichen $R_f$-Wert von $R_f$ ≅ 0,60 aufweisen, werden vereinigt und durch Abdestillieren vom Lösungsmittel befreit. Der Rückstand wird aus Methanol/Diisopropyläther umkristallisiert und ergibt 5,2 g 21-Desoxy-dexamethason-17-n-propylcarbonat-21-chlorid vom Schmp. 190°C.

| | |
|---|---|
| Charakt. IR-Banden: | 3430, 1730, 1655, 1610, 1285, 1260 $cm^{-1}$ |
| Massenspektrum: | $M^{\oplus}$ = 496 |
| DC: | $R_f$ ≅ 0,63 (keine Nebenflecke) |
| UV: | λmax. = 239 nm; ε = 15600 |

b) Zum gleichen Verfahrensprodukt mit den gleichen Daten wie unter Beispiel 32 a) angegeben, gelangt man, wenn man anstelle des Dexamethason-17-n-propylcarbonat-21-p-chlorbenzolsulfonats eine äquimolekulare Menge von Dexamethason-17-n-propylcarbonat-21-toluolsulfomat in die Reaktion einsetzt und in gleicher Weise wie unter Beispiel 32 a) angegeben, umsetzt und aufarbeitet.

Beispiel 33

Wird in die Reaktion gemäß Beispiel 32 a) anstelle des Lithiumchlorids eine äquimolekulare Menge Lithiumbromid eingesetzt und in gleicher Weise wie dort angegeben, umgesetzt, so erhält man nach dem Eingießen des Reaktionsansatzes in Wasser, Abfiltrieren des Niederschlags, Waschen mit Wasser und Trocknen des Niederschlags über Phosphorpentoxyd im Hochvakuum bei 70°C 4,8 g 21-Desoxy-dexamethason-17-n-propylcarbonat-21-bromid vom Schmp. 175° – 180°C.

| | |
|---|---|
| IR-Banden: | 3430, 1730, 1660, 1610, 1270 $cm^{-1}$ |
| UV: | λmax. = 238 nm; ε = 15700 |

Beilsteinprobe positiv

Beispiel 34

Eine Lösung von 2,3 g Dexamethason-17-n-propylcarbonat-21-p-chlorbenzolsulfonat in 46 ml absol. Äthylmethylketon wird mit 800 mg Natriumjodid versetzt. Nach 2stündigem Rückflußkochen wird in 450 ml Kochsalz enthaltendes Wasser eingerührt, wobei ein Niederschlag ausfällt, der abfiltriert wird. Nach dem Waschen mit Wasser, Trocknen im Vakuum über $P_2O_5$, wird der Niederschlag mit Diäthyläther digeriert. Man erhält 2,0 g 21-Desoxy-dexamethason-17-n-propylcarbonat-21-jodid.

Charakt. IR-Banden: 3420, 1730, 1660, 1610, 1270 $cm^{-1}$
DC: $R_f \cong 0{,}50$ (Laufmittel = Toluol : Methanol : Aceton = 80 : 20 : 5)
UV: $\lambda max. = 238$ nm; $\varepsilon = 14900$
Beilsteinprobe positiv

Beispiel 35

In gleicher Weise wie in Beispiel 4 beschrieben, werden 1 g Dexamethason-17-n-propylcarbonat-21-p-chlorbenzolsulfonat umgesetzt und aufgearbeitet. Man erhält das 21-Desoxy-dexamethason-17-n-propylcarbonat-21-fluorid.

UV: $\lambda max. = 238$ nm; $\varepsilon = 15100$

Herstellung von Dexamethason-17-n-propylcarbonat-21-p-chlorbenzolsulfonat

Zu einer Lösung von 3 g Dexamethason-17-n-propylcarbonat in 35 ml abs. Aceton und 12 ml abs. Pyridin werden bei 0°C 2,6 g p-Chlorbenzolsulfonsäurechlorid zugegeben. Nach 20 Stunden Rühren bei 0° bis 22°C (allmählich Temperatur ansteigen lassen) wird in Wasser eingegossen, das ausgefallene Öl abfiltriert und dieses mit Methylenchlorid aufgenommen, dann wird gewaschen und das Lösungsmittel im Vakuum eingeengt.

Man erhält 3,8 g Dexamethason-17-n-propylcarbonat-21-p-chlorbenzolsulfonat als Schaum vom Erweichungspunkt 87°C.

Massenspektrum: $M^{\oplus} = 652$
IR: 3430, 1730, 1660, 1620–1600, 1370, 1260, 1180 $cm^{-1}$
UV: $\lambda max. = 232$ nm; $\varepsilon = 27900$

Beispiel 36

21-Deoxy-21-fluor-dexamethason-17-äthylcarbonat

a) Eine Lösung von 0,5 g Dexamethason-17,21-diäthylorthocarbonat in 5 ml abs. Dimethylformamid werden nach Zugabe von 0,7 ml Trimethylsilylfluorid 3 Stunden lang bei 25° gerührt. Anschließend wird in 30 ml Wasser eingerührt und mit Methylenchlorid extrahiert. Nach mehrmaligem Waschen der vereinigten Methylenchloridextrakte mit Wasser wird die organische Phase i.V. zur Trocknung eingeengt. Der Destillationsrückstand wird in sehr wenig Methylenchlorid gelöst und an einer Säule aus 20 g Kieselgel mit Methylenchlorid als Elektionsmittel wie üblich chromatographiert. Es wurden nach dem Umkristallisieren aus Aceton/Hexan 160 mg 21-Desoxy-21-fluor-dexamethason-17-äthylcarbonat vom Smp. 167–170° erhalten.
b) 500 mg Dexamethason-17-äthylcarbonat-21-methansulfonat wurden zu einer vorher bereits 35 Minuten bei Raumtemperatur gerührten Suspension von 73 mg 18-Krone-6 und 95 mg Kaliumfluorid in 4 ml Acetonitril gegeben. Dann wird in einer $N_2$-Atmosphäre 8 Stunden lang bei 80° gerührt. Nach dem Abkühlen wird das Reaktionsgemisch in 20 ml Wasser eingerührt und wie unter a) beschrieben aufgearbeitet. Es werden 140 mg 21-Desoxy-21-fluor-dexamethason-17-äthylcarbonat erhalten. das mit dem unter a) erhaltenen Produkt identisch ist.

Beispiel 37

Werden in Beispiel 18 a) anstelle von Dexamethason-17-äthylcarbonat-21-p-chlorbenzol-sulfonat das 17-Äthylcarbonat oder das 17-n-Propylcarbonat oder das 17-n-Butylcarbonat des Prednison-, Cortisol-, Cortison-, 6α-Methyl-prednisolon-, Betamethason-, Beclomethason-, 6α-Fluor-dexamethason-, 6α-Fluor-prednisolon-, 16α- oder 16β-Methylprednisolon-, 6α, 16α- oder β-Dimethylprednisolon- bzw. 9α-Chlor-prednisolon-21-p-chlorbenzol-sulfonats in die Reaktion eingesetzt und ebenso zur

Reaktion gebracht, aufgearbeitet und isoliert, wie dort angegeben wurde, so erhält man die entsprechenden 21-Chlor-21-desoxy-17-alkyl-carbonate-corticoide der angegebenen Corticosteroide.

## Beispiel 38

Werden in Beispiel 19) anstelle von Dexamethason-17-äthylcarbonat-21-p-chlorbenzol-sulfonat das 17-Äthylcarbonat oder das 17-n-Propylcarbonat oder das 17-n-Butylcarbonat des Prednison-, Cortisol-, Cortison-, 6α-Methyl-prednisolon-, Betamethason-, Beclomethason-, 6α-Fluor-dexamethason-, 6α-Fluor-prednisolon-, 16α- oder 16β-Methylprednisolon-, 6α, 16α- oder β-Dimethylprednisolon- bzw. 9α-Chlorprednisolon-21-p-Chlorbenzolsulfonats in die Reaktion eingesetzt und ebenso zur Reaktion gebracht, aufgearbeitet und isoliert, wie dort angegeben wurde, so erhält man die entsprechenden 21-Brom-21-desoxy-17-alkylcarbonat-corticoide der angegebenen Corticosteroide.

## Beispiel 39

Werden in Beispiel 20) anstelle von Dexamethason-17-äthylcarbonat-21-p-chlorbenzol-sulfonat das 17-Äthylcarbonat oder das 17-n-Propylcarbonat oder das 17-n-Butylcarbonat des Prednison-, Cortisol-, Cortison-, 6α-Methyl-prednisolon-, Betamethason-, Beclomethason-, 6α-Fluor-dexamethason-, 6α-Fluor-prednisolon-, 16α- oder 16β-Methylprednisolon-, 6α, 16α- oder β-Dimethylprednisolon- bzw. 9α-Chlorprednisolon-21-p-chlorbenzol-sulfonats in die Reaktion eingesetzt und ebenso zur Reaktion gebracht, aufgearbeitet und isoliert, wie dort angegeben wurde, so erhält man die entsprechenden 21-Jod-21-desoxy-17-alkylcarbonat-corticoide der angegebenen Corticosteroide.

## Beispiel 40

### 21-Deoxy-21-chlor-dexamethason-17-äthylcarbonat und Analogpräparate

Eine Lösung von 0,5 g Dexamethason-17,21-diäthylorthocarbonat in 5 ml abs. Dimethylformamid oder Acetonitril werden nach Zugabe von 1 ml Trimethylsilylchlorid 16 Stunden lang bei 25° C gerührt. Anschließend wird in 30 ml Wasser eingerührt und mit Methylenchlorid extrahiert. Nach mehrmaligem Waschen der vereinigten Methylenchloridextrakte mit Wasser wird die organische Phase i. V. zur Trocknung eingeeingt. Der Destillationsrückstand wird in sehr wenig Methylenchlorid gelöst und an einer Säule aus 20 g Kieselgel mit Methylenchlorid als Elektionsmittel wie üblich chromatographiert. Es wurden nach dem Umkristallisieren aus Aceton/Hexan 170 mg 21-Desoxy-21-chlor-dexamethason-17-äthylcarbonat mit den gleichen Daten, wie unter Beispiel 18 a) beschrieben, erhalten.

Wird in die Reaktion eine entsprechende Menge Dexamethason-17,21-di-n-propylorthocarbonat eingesetzt und in gleicher Weise die Reaktion mit Trimethylsilylchlorid durchgeführt und aufgearbeitet, wie eben beschrieben, so erhält man nach dem Kristallisieren das 21-Desoxy-21-chlor-dexamethason-17-n-propylcarbonat mit dem gleichen Schmp., wie in Beispiel 22b) angegeben.

In gleicher Weise erhält man, wenn man in die Reaktion statt der Dexamethason-17,21-dialkylorthocarbonate jeweils 0,5 g des Prednisolon-17,21-diäthyl- bzw. des -di-n-propylcarbonats einsetzt, nach analoger Reaktionsführung, Aufarbeitung und Isolierung das 21-Desoxy-21-chlor-prednisolon-17-äthylcarbonat bzw. das 21-Desoxy-21-chlor-prednisolon-17-n-propylcarbonat, die jeweils in allen Daten mit den entsprechenden Verfahrensprodukten, die nach Beispiel 1 bzw. Beispiel 5a) erhalten werden, identisch sind.

## Beispiel 41

Zu einer Lösung von 2,5 g 21-Desoxy-dexamethason-17-äthylcarbonat-21-chlorid, hergestellt nach Beispiel 18 a) in 75 ml Aceton p. A. werden bei 0° C und unter Rühren 2 ml einer $CrO_3$-Oxydationslösung (Herstellung: 13,36 g $CrO_3$ werden in 30 ml Wasser gelöst und unter Eiskühlung läßt man dazu 11,5 ml konz. Schwefelsäure zutropfen, anschließend füllt man auf 50 ml auf) zugetropft. Nach 1 Std. Rühren bei 0° C und 1,5 Std. Rühren bei 20° C gießt man das Reaktionsgemisch in Wasser ein, das eine zur Neutralisation erforderliche Menge Pyridin enthält, extrahiert mehrmals mit Methylenchlorid, wäscht mit Wasser, trocknet und engt im Vakuum ein. Der erhaltene Schaum wird aus Aceton/Diisopropyläther umkristallisiert und ergibt 2,0 g 21-Desoxy-11-dehydro-dexamethason-17-äthylcarbonat-21-chlorid vom Schmp. 214° C.

IR: 1720–1740, 1660, 1625, 1280, 1260 $cm^{-1}$, keine Bande im Bereich um 3420 $cm^{-1}$ (OH) mehr vorhanden!
UV: $\lambda$max. = 238 nm; $\varepsilon$ = 15200

Werden anstelle des 21-Desoxy-11-dehydro-dexamethason-17-äthyl-21-chlorids jeweils die in den vorausgegangenen Beispielen dargestellten Corticoid-17-alkylcarbonate, die in 11-Stellung eine Hydroxylgruppe und in 21-Stellung entweder ein Halogenatom (F, Cl, Br, J) oder eine Alkyl- bzw. Arylsulfonsäureestergruppe aufweisen, in die eben beschriebene Oxydationsreaktion eingesetzt, so erhält man nach analoger Reaktionsführung und Aufarbeitung die entsprechenden 21-Desoxy-11-dehydro-corticoid-17-alkylcarbonat-21-halogenide bzw. -21-alkylsulfonsäureester bzw. -21-arylsulfonsäureester.

**Patentansprüche**

1. Corticoid-21-halogen-17-alkylcarbonate der allgemeinen Formel I oder II

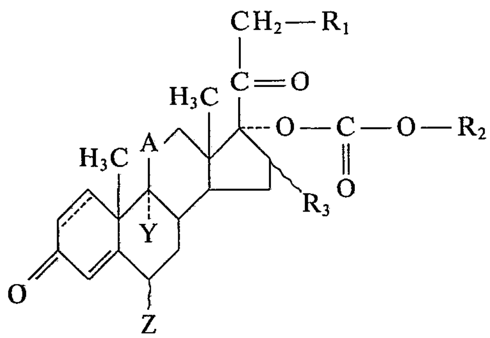

(I)

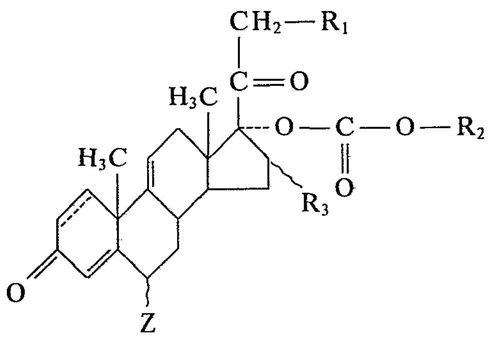

(II)

in welcher bedeuten A die Gruppierungen

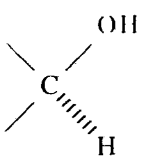

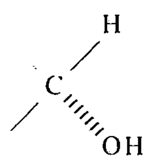

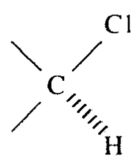

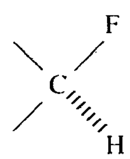

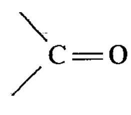

Y Wasserstoff, Fluor oder Chlor,
Z Wasserstoff, Chlor, Fluor oder Methyl,
$R^1$ Fluor, Chlor, Brom, Jod,
$R_2$ Alkyl mit 1 bis 10 C-Atomen,
$R_3$ Wasserstoff, $\alpha$- oder $\beta$-ständiges Methyl, Fluor oder eine gegebenenfalls durch ein oder zwei Fluoratome substituierte Methylengruppe,

wobei in 1,2-Stellung eine zusätzliche Doppelbindung vorhanden sein kann.

2. Verfahren zur Herstellung von Corticoid-17-alkylcarbonaten gemäß Anspruch 1, dadurch gekennzeichnet, daß man

a) ein Steroid-21-alkyl- bzw. arylsulfonat-17-alkylcarbonat der Formel I oder II in der $R_2$, $R_3$, A, Y und Z die in Anspruch 1 genannten Bedeutungen haben, $R_1$ jedoch einen Rest der Formel III

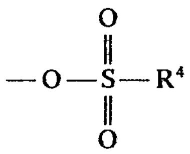

(III)

darstellt, worin $R^4$ $C_1-C_4$-Alkyl, Phenyl, Methylphenyl-, Äthylphenyl, Fluor, Brom- oder Chlorphenyl oder Nitrophenyl bedeutet, mit einer Halogen-Anionen liefernden Verbindung in Gegenwart von inerten organischen Lösungsmitteln umsetzt, wobei gegebenenfalls auch nach dem Phasentransverfahren wäßrige Medien benutzt werden können, oder

b) ein Corticosteroid-17,21-dialkylorthocarbonat der Formel IV oder V

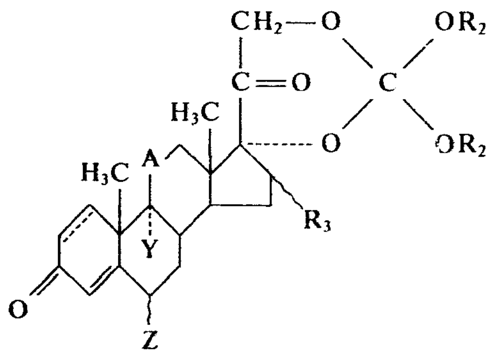

(IV)

oder

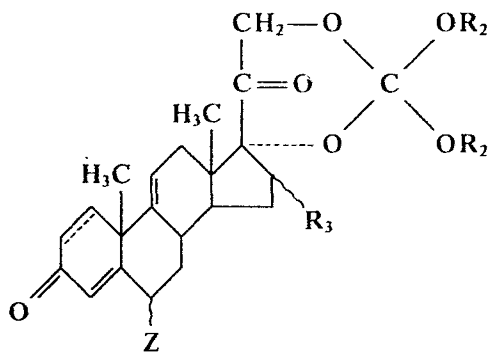

(V)

in der A, Y, Z, $R_2$ und $R_3$ die in Anspruch 1 angegebene Bedeutung haben, mit Halogen abgebenden anorganischen oder organischen Säurehalogenid-Verbindungen oder mit Triphenylmethylchlorid in inerten organischen Lösungsmitteln umsetzt.

3. Arzneimittel, gekennzeichnet durch einen Gehalt an einer Verbindung gemäß Anspruch 1.

## Claims

1. Corticoid-21-halogeno-17-(alkyl carbonates) of the formulae I or II

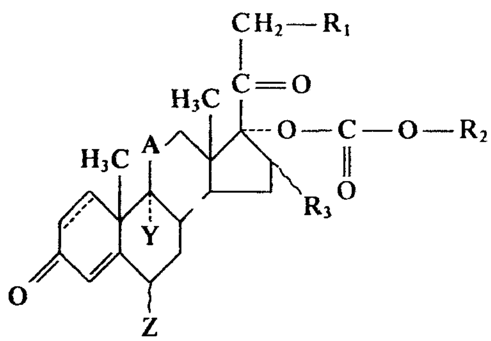

(I)

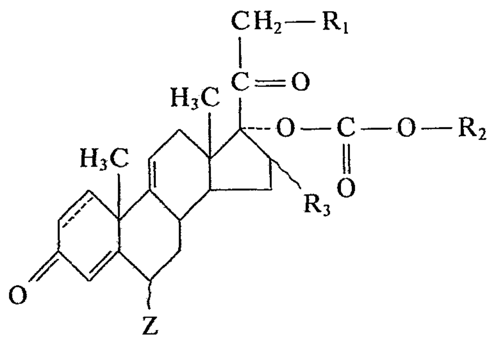

(II)

in which A denotes the groupings

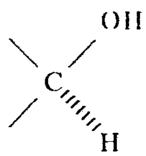
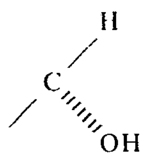
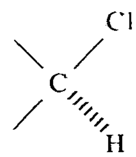
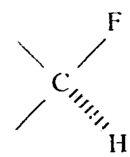
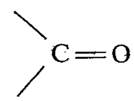

Y denotes hydrogen, fluorine or a chlorine atom,
Z denotes hydrogen, chlorine, fluorine or a methyl group,
$R_1$ denotes fluorine, chlorine, bromine or iodine,
$R_2$ denotes alkyl radical having from 1 to 10 carbon atoms,
$R_3$ denotes hydrogen, methyl in $\alpha$- or $\beta$-position, fluorine or a methylene group optionally substituted by one or two fluorine atoms,

optionally with additional double bonds in 1,2-position.

2. A process for the manufacture of a corticoid-17-(alkyl carbonate) as claimed in claim 1, which comprises

a) reacting a steroid-21-alkyl- or -aryl-sulfonate-17-(alkyl carbonate) of the formulae I or II in which $R_2$, $R_3$, A, Y and Z are difined as claimed in claim 1, however, $R_1$ denotes a radical of the formula III

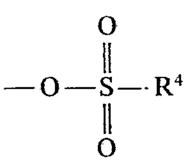

(III)

in which $R^4$ is $C_1-C_4$-alkyl, phenyl, methylphenyl, ethylphenyl, fluorophenyl, bromophenyl, chlorophenyl or nitrophenyl, with a compound donating halogen anions, in the presence of inert organic solvents, and/or optionally aqueous media according to the phase transfer process, or

b) reacting a corticosteroid-17,21-(dialkyl orthocarbonate) of the formulae IV or V

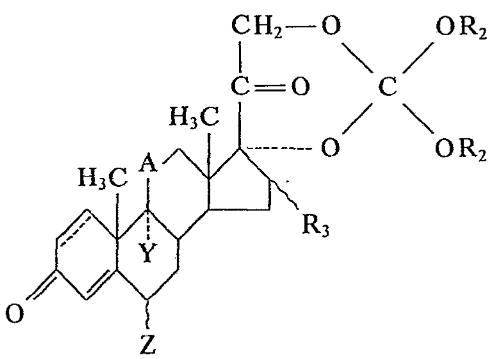

(IV)

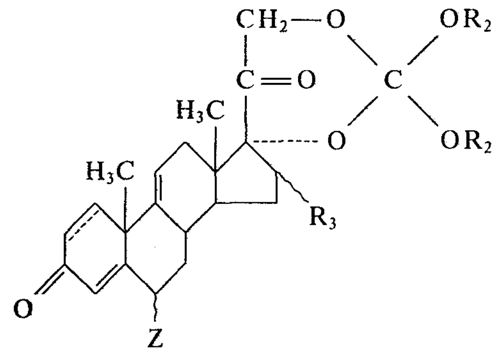

(V)

in which A, Y, Z, $R_2$ and $R_3$ are defined as claimed in claim 1, with halogen-donating inorganic or organic acid halides or with triphenylmethyl chloride in inert organic solvents.

3. Medicament which comprises an amount of a compound claimed in claim 1.

**Revendications**

1. Corticoïde-21-halogèno-17-carbonates d'alkyle répondant aux formules générales I ou II

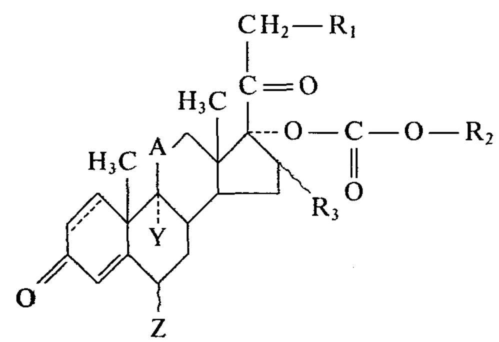

(I)

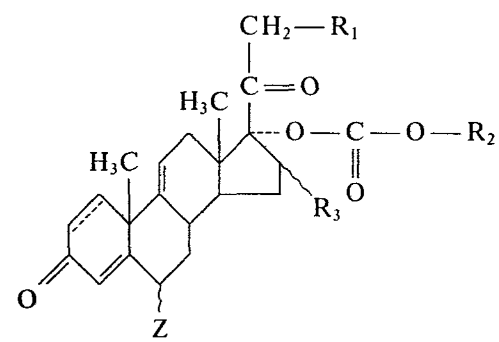

(II)

dans lesquelles A désigne les groupes

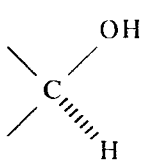 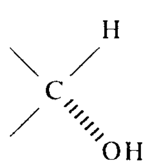 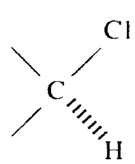 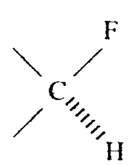 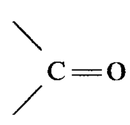

Y l'hydrogène, le fluor ou le chlore,
Z l'hydrogène, le chlore, le fluor ou un groupe méthyle,
$R_1$ le fluore, le chlore, le brome ou l'iode,
$R_2$ un groupe alkyle en $C_1$ à $C_{10}$,
$R_3$ l'hydrogène, un groupe méthyle en $\alpha$ ou en $\beta$, le fluor ou un groupe méthylène éventuellement substitué par 1 ou 2 atomes de fluor, une double liaison supplémentaire pouvant être présente en position 1,2.

2. Procédé de préparation de corticoïde-17-carbonates d'alkyle suivant la revendication 1, caractérisé en ce que

a) on fait réagir un stéroïde-21-alkyle- ou arylsulfonate-17-carbonate d'alkyle répondant aux formules I ou II dans lesquelles $R_2$, $R_3$, A, Y et Z ont les significations indiquées dans la revendication 1, mais $R_1$ représente un radical répondant à la formule III

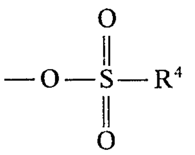

(III)

dans laquelle $R^4$ désigne un groupe alkyle en $C_1$ à $C_4$, phényle, méthylphényle, éthylphényle, fluoro-, bromo- ou chlorophényle, ou nitrophényle, avec un composé fournissant des anions halogènes, en présence de solvants organiques inertes, des mulieux aqueux pouvant le cas échéant être utilisés selon le procédé de transfert de phase, ou

b) on fait réagir un corticostéroïde-17,21-orthocarbonate de dialkyle répondant aux formules générales IV ou V

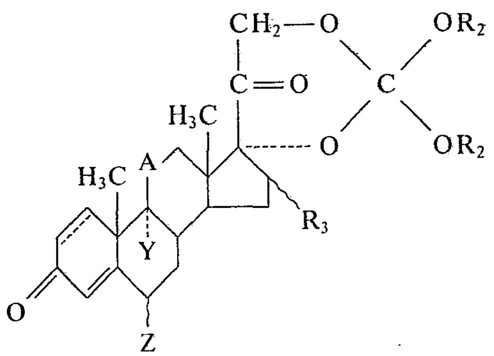

(IV)

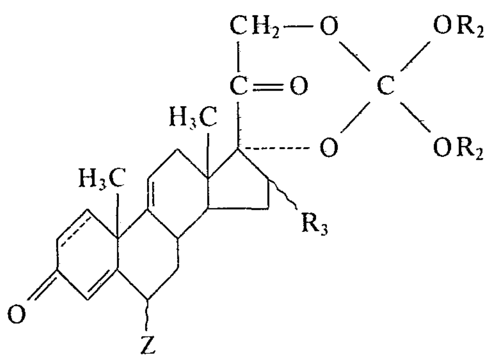

(V)

dans lesquelles A, Y, Z, $R_2$ et $R_3$ ont la signifacation indiquée dans la revendication 1, avec des halogénures d'acides minéraux ou organiques fournissant des halogènes, ou avec du chlorure de triphénylméthyle dans des solvants organiques inertes.

3. Médicament caractérisé en ce qu'il contient un composé suivant la revendication 1.